# EUROPEAN PATENT SPECIFICATION

(11) **EP 2 588 854 B1**
(45) Date of publication and mention of the grant of the patent: **31.05.2017**
(21) Application number: 10854225.9
(22) Date of filing: 30.06.2010
(51) Int. Cl.: G01N 33/574, G01N 33/00, C12Q 1/02, C12Q 1/54, G01N 33/66

(54) **METHOD TO MEASURE THE METABOLIC RATE OR RATE OF GLUCOSE CONSUMPTION OF CELLS OR TISSUES WITH HIGH SPATIOTEMPORAL RESOLUTION USING A GLUCOSE NANOSENSOR**
VERFAHREN ZUR MESSUNG DER VERSTOFFWECHSELUNGSRATE ODER GLUCOSEVERBRAUCHSRATE VON ZELLEN ODER GEWEBE MIT HOHER RÄUMLICH-ZEITLICHER AUFLÖSUNG ANHAND EINES GLUCOSENANOSENSORS
MÉTHODE DE MESURE DU TAUX MÉTABOLIQUE OU DU TAUX DE CONSOMMATION DU GLUCOSE PAR DES CELLULES OU DES TISSUS À UNE RÉSOLUTION SPATIOTEMPORELLE ÉLEVÉE À L'AIDE D'UN NANOCAPTEUR DE GLUCOSE

(43) Date of publication of application: 08.05.2013
(73) Proprietor: Centro De Estudios Cientificos De Valdivia, Valdivia (CL); CARNEGIE INSTITUTION OF WASHINGTON, Washington, D.C. 20005 (US)
(72) Inventor: BARROS OLMEDO, Luis, Felipe, Valdivia (CL); BITTNER HOFMANN, Carla, Ximena, Valdivia (CL)
(74) Representative: Carvajal y Urquijo, Isabel
(86) International application number: PCT/US2010/040643
(87) International publication number: WO 2012/002963

(56) References cited:
- WO-A2-2007/046786
- US-A1- 2005 197 311
- US-A1- 2007 020 181
- US-A1- 2007 087 401
- US-A1- 2010 037 329
- TAKANAGA HITOMI ET AL: "GLUT1 and GLUT9 as major contributors to glucose influx in HepG2 cells identified by a high sensitivity intramolecular FRET glucose sensor.", BIOCHIMICA ET BIOPHYSICA ACTA APR 2008, vol. 1778, no. 4, April 2008 (2008-04), pages 1091-1099, XP002713883, ISSN: 0006-3002
- OKUMOTO ET AL: "Imaging approach for monitoring cellular metabolites and ions using genetically encoded biosensors", CURRENT OPINION IN BIOTECHNOLOGY, LONDON, GB, vol. 21, no. 1, 1 February 2010 (2010-02-01), pages 45-54, XP026969181, ISSN: 0958-1669 [retrieved on 2010-02-16]
- WOOD ET AL.: 'Hypoxia Increases Expression Of Selective Facilitative Glucose Transporters (GLUT) and 2-Deoxy-D-Glucose Uptake In Human Adipocytes.' BIOCHEMICAL AND BIOPHYSICAL RESEARCH COMMUNICATIONS vol. 361, 2007, pages 468 - 473, XP027016339
- FEHR MARCUS ET AL: "In vivo imaging of the dynamics of glucose uptake in the cytosol of COS-7 cells by fluorescent nanosensors", JOURNAL OF BIOLOGICAL CHEMISTRY, AMERICAN SOCIETY FOR BIOCHEMISTRY AND MOLECULAR BIOLOGY, US, vol. 278, no. 21, 23 May 2003 (2003-05-23) , pages 19127-19133, XP002553883, ISSN: 0021-9258, DOI: 10.1074/JBC.M301333200 [retrieved on 2003-03-20]
- CARLA X. BITTNER: "High resolution measurement of the glycolytic rate", FRONTIERS IN NEUROENERGETICS, vol. 2, 1 January 2010 (2010-01-01), XP055169934, ISSN: 1662-6427, DOI: 10.3389/fnene.2010.00026
- SCOTT A JOHN ET AL: "Dynamic modulation of intracellular glucose imaged in single cells using a FRET-based glucose nanosensor", PFLÜGERS ARCHIV - EUROPEAN JOURNAL OF PHYSIOLOGY, SPRINGER, BERLIN, DE, vol. 456, no. 2, 11 December 2007 (2007-12-11), pages 307-322, XP019630218, ISSN: 1432-2013

## Description

### FIELD OF THE INVENTION

The present invention is a new method to measure the metabolic rate or rate of glucose consumption of cells or tissues with high spatiotemporal resolution using a glucose nanosensor. This method can be applied for the screening of molecules with pharmacological potential, determination of glucose rate of cancerous cells, tissue physiology and biochemistry research.

### BACKGROUND OF THE INVENTION

The metabolic rate is the speed at which the body burns fuel and is sensitive to physiological activity, hormones, stress, aging and malignant transformation. Inside tissues, every cell is characterized by a specific metabolic rate, ranging from low for quiescent cells like skin fibroblasts to very high for some cells in epithelia and muscle. The metabolic rate of an individual cell can also vary through time: for instance, adipocytes increase their rate of glucose uptake by up to ten-fold in response to insulin, whereas neurons may raise their energy demand by larger factors in response to electrical stimulation. The metabolic rate is also affected by aging and disease. For example cancer cells show higher metabolic rates than their surrounding tissue, a phenomenon involved in tumor progression and instrumental for the purposes of diagnosis, staging and prognosis of this disease ^{1,2}.

There are no available methods to measure the metabolic rate in single cells. More specifically, current and common techniques to measure the metabolic rate using radioactive isotopes can not resolve single cells and have poor temporal resolution, which hampers the study of complex tissues or fast phenomena.

Cell populations and tissues are usually studied by measuring the uptake of radioactive metabolites. In a typical *in vitro* experiment, a million cells in a culture dish are exposed for 20 minutes to radiolabeled deoxyglucose, which is phosphorylated by hexokinase, so that the radioactivity trapped inside the cells is assumed to be proportional to the metabolic rate. For *in vivo* experiments, the tracer is given intravenously, and after a period of 20 minutes or more, the radioactivity accumulated in the tissue is detected by autoradiography or non-invasively by Fluorodeoxyglucose (FDG)-PET scanning, techniques that have found wide application in functional mapping of the brain and for the detection of tumors. Useful as they are, deoxyglucose uptake, deoxyglucose autoradiography and FDG-PET scanning have their limitations. Firstly, they do not use glucose but an analog, which is not handled by the cell in the same way. Secondly, uptake of glucose analogs is not just determined by metabolic rate but also depends on the usually unknown properties of the glucose transporters that mediate their entry into the cell. Thirdly, they offer low spatiotemporal resolution, which precludes resolving the contribution of individual cells or detecting rapid phenomena; and finally, they are relatively insensitive and require isotope manipulation, which makes them inadequate for the purposes of high-throughput analysis.

Furthermore, as presently known, all patent applications related to metabolic rate determination are directed to the measurement of glucose, but no patent application has addressed the issue of metabolic rate determination. In particular, US patent application 20050197311 where compositions and methods for measuring intracellular glucose are described. This application uses nucleic acid constructs comprising a glucose-regulated mRNA instability element. This instability element is coupled to a reporter vector which will be downregulated in the presence of high glucose concentrations. The limitation of this method is the delay in protein expression and therefore, the method is not suitable for applications where a good time resolution is required, as in the case of determining the metabolic rate of single cells or tissues. The present invention allows temporal resolution of minutes and even seconds compared to the temporal resolution of hours achieved by the description found in US patent application 20050197311.

US patent application 20050118726 describes a sensor comprising different protein domains based on a fusion protein comprising fluorescent domains. It also describes potential devices for the detection of glucose concentration. Nevertheless, it does refer to the temporal resolution of the sensor and it does not address the issue of metabolic rate determination.

Fluorescence allows estimation of metabolite concentration with high sensitivity and spatiotemporal resolution. In addition to NAD(P)H autofluorescence ³, the increasing availability of DNA-encoded probes has made it possible to measure the concentration of several metabolites using fluorescence ^{4,5}. Whereas metabolite concentrations are interesting *per se,* the presence of homeostatic mechanisms in living cells makes steady-state concentrations of little value for the purposes of flux prediction.
It is possible nowadays to measure the concentration of glucose in single cells using FRET nanosensors ^{4,6,7}.

International patent application WO2006006166 describes a glucose sensor based on yellow fluorescent protein (YFP) and cyan fluorescente protein (CFP), US patent 7,432,353 describes a maltose sensor comprising different moieties based on fluorescent proteins such as enhanced yellow, cyan, blue and green fluorescent proteins and red fluorescent protein. International patent application WO2007046786 describes a glucose fluorescent sensor. Although all these patent applications mention a fluorescent glucose or maltose sensor, none of them addresses the issue of determination of the metabolic rate in single cells or tissues.

However, as with any other metabolite, the concentration of glucose in the steady-state is not informative about flux through the metabolic pathway. The present invention provides a method to approach glucose flux.

In the present invention, the inconvenient of measuring glucose concentration in steady-state has been circumvented by interrupting the steady-state, while measuring the concentration of glucose with a DNA-encoded nanosensor. In particular, the method of the present invention allowed for the first time to observe that astrocytic glycolysis can be activated by neuronal signals within seconds, supporting central roles for astrocytes in neurometabolic and neurovascular coupling in the brain. It was also possible to make a direct comparison of metabolism in neurons and astrocytes lying in close proximity, opening the way to a high resolution characterization of brain energy metabolism. Single-cell metabolic rates have also been measured in fibroblasts, adipocytes, myoblasts and tumor cells, evidencing metabolic heterogeneity, even for cell lines. The method of the present invention allows the investigation of tissue metabolism at the single cell level and is readily adaptable for high-throughput analysis using microtiter plates.

### BRIEF DESCRIPTION OF THE INVENTION

The present invention addresses the issues related to glucose measurements to determine the metabolic rate arising from the limitations imposed by steady-state, by disrupting the steady-state. The disruption of the steady-state to perform analyte measurements is not obvious considering the previous art, since all the usual and current methods rely on the assumption of steady-state, and therefore, providing the conditions to keep the steady-state, instead of disrupting it. The disruption of steady-state will only provide useful results and information if the method, analyte sensor, and proper interpretation of data are achieved.

The invention comprises a method for the measurement of glucose metabolic rate. The method can be applied to single cells or cell populations, cells in suspension or adherent, to a cell culture, a tissue culture, a mixed cell culture, a tissue explant, or it can also be applied to animal tissues *in vivo.* The method comprises the expression of a suitable glucose sensor in individual cells. The glucose sensor should be able to monitor the glucose concentration in real-time. Suitable glucose sensors should provide an easy to read signal in a glucose concentration-dependent manner and be insensitive to other molecules commonly present in cells. The expression, presence or degradation of the sensor should not interfere significantly with the cell metabolism. A non limiting example of such a sensor would be the one described in patent application WO2007046786.

The glucose sensor should be expressed in single cells or cell populations, cells in suspension or adherent, in a cell culture, a tissue culture, a mixed cell culture, a tissue explant, or in animal tissues *in vivo.* When using a genetically-encoded glucose sensor, the gene expression can be attained by any suitable method to transfer the sensor gene information to the host cell. Examples of gene transfer methodologies are plasmid transfer for instance using liposomal delivery, virus transfer and transgenesis.

Once the sensor is expressed in single cells or cell populations, cells in suspension or adherent, in a cell culture, a tissue culture, a mixed cell culture, a tissue explant, or in animal tissues *in vivo,* the sensor is calibrated according to pre-established conditions.

With the information obtained in the calibration step, the determination of the metabolic rate is carried out by disrupting the flux of glucose, which is normally maintained in a steady-state. The disruption of the steady-state is obtained by changing the extracellular concentration of glucose to a level which is inferior to the intracellular glucose level.

Once the disruption of the steady-state is obtained, the registry of data produced by the sensor allows the determination of the metabolic rate.

Summarizing, the method of the present invention comprises the following steps:
- Providing a system for the measurement of glucose metabolic rate. The system can be single cells or cell populations, cells in suspension or adherent, a cell culture, a tissue culture, a mixed cell culture, a tissue explant, or animal tissues *in vivo;*
- Expressing a suitable glucose sensor in individual cells. The gene expression single cells or cell populations, cells in suspension or adherent, in a cell culture, a tissue culture, a mixed cell culture, a tissue explant, or in animal tissues *in vivo;*
- Calibrating the sensor in the controlled conditions;
- Disrupting the steady-state of glucose entering the cell by exposing the extracellular space to a variation in the concentration of glucose (ETM: Equilibrium Transport Method);
- Recording the output from the sensor and calculate the corresponding glucose concentration at different times; and
- Determining the glucose metabolic rate.

The preferred type of cells or tissue are those that express equilibrative glucose transporters and do not express Na⁺-dependent glucose transporters, which include neurons, astrocytes, muscle cells, adipocytes, liver cells, pancreatic cells, fibroblasts, stem cells, blood cells, endothelial cells, and most mammalian cells types.

### BRIEF DESCRIPTION OF THE DRAWINGS

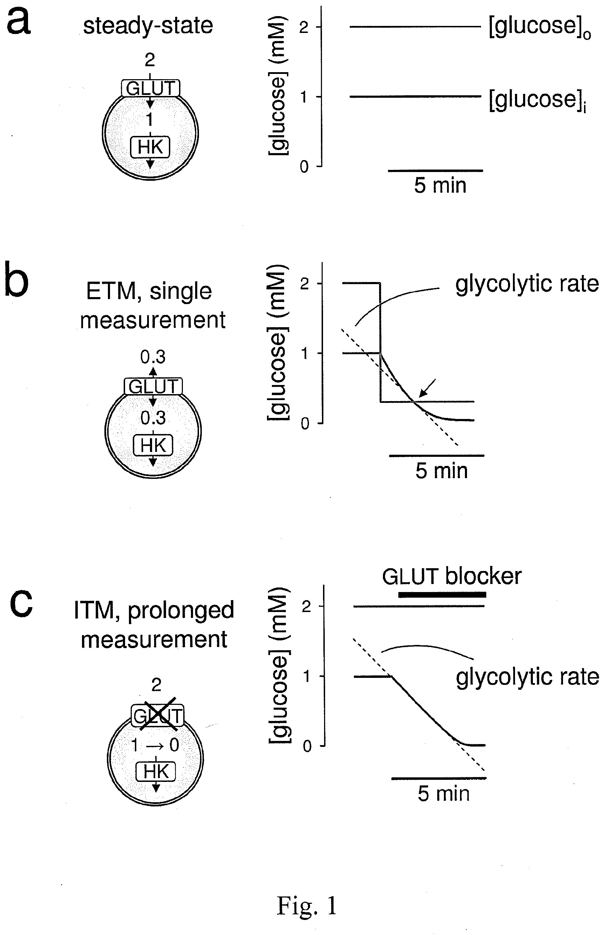

Figures 1(a)-(c) schematically illustrate the method of the invention, identified as ETM, and the use of an inhibitor of the glucose transporter GLUT the disruption of the steady-state, identified as ITM.
Figures 2(a)-(c) demonstrate ETM variant of the method used in astrocytes.
Figures 3(a)-(f) demonstrate ITM variant of the method in astrocytes.
Figures 4(a) and 4(b) illustrate temporal resolution involving activation of astrocytic glycolysis by neuronal signals using ITM.
Figures 5(a)-(d) illustrate spatial resolution involving simultaneous measurement of glycolytic rate in astrocytes and neurons.
Figures 6 (a)-(f) comprise graphs showing the heterogeneity of glycolytic rates in various cells determined by using ITM.
Figure 7 (a)-(d) Calibration of the FLII12Pglu600Δµ6 nanosensor. The nanosensor was calibrated in astrocytes (a), 3T3-L1 fibroblasts (b), C2C21 myoblasts (c) and HeLa cells (d) by exposing the cultures to increasing concentrations of glucose (0-2 mM) after full inhibition of glycolysis by preincubation with iodoacetic acid for 30 min. Data are from at least 7 cells from three experiments for each cell type. The saturation parameters were obtained by fitting a rectangular hyperbola to the data using non-linear regression.

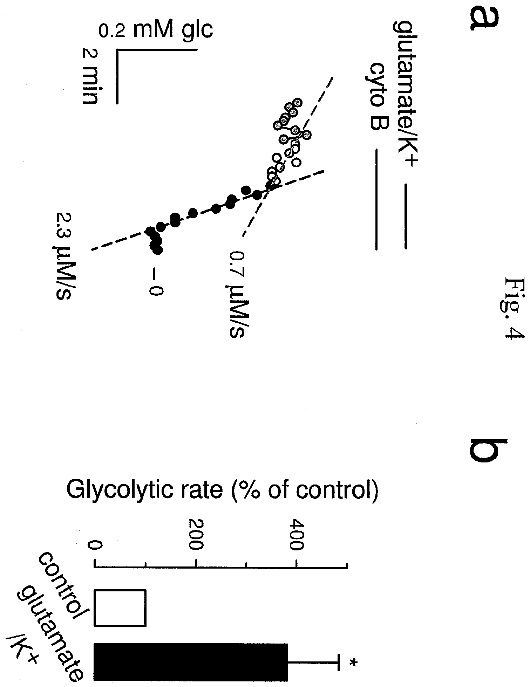

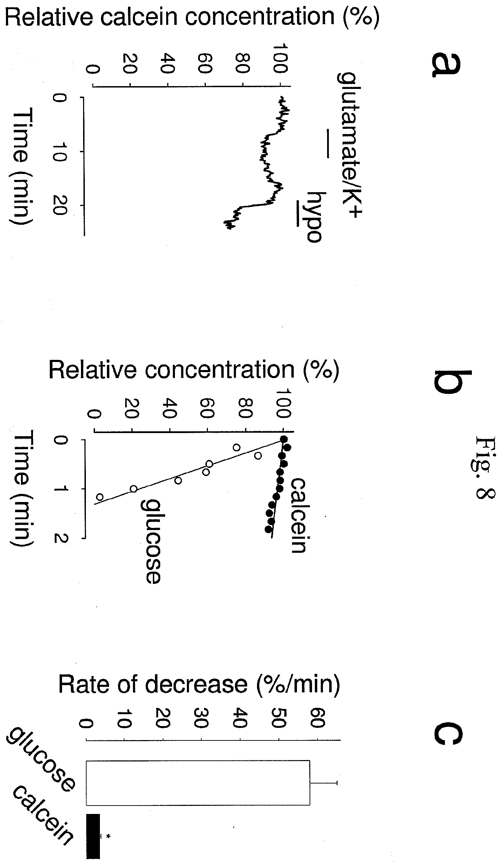

Figure 8 (a)-(c) Effect of glutamate/K⁺ on astrocytic cell volume. a) A calcein-loaded cell was first exposed to 50 µM glutamate/ 15 mM K⁺ and then to a solution in which NaCl had been reduced to make the solution 30% hypotonic (hypo). Relative calcein concentration was calculated from calcein fluorescence using the response to hypotonicity as a calibration factor. b) The initial time course of the response to glutamate/K+ shown in A (calcein) is plotted together with an example of relative decrease in glucose concentration elicited by K⁺ (glucose, same data as shown in Fig. 4a). c) Initial rates of decrease are given for a series of experiments with 50 µM glutamate/15 mM K⁺ for glucose (n=25 cells in five experiments) and calcein (n=20 cells in three experiments).

### DETAILED DESCRIPTION OF THE INVENTION

As mentioned before, the method of the present invention is directed to the measurement of the metabolic rate in different systems. In particular, the method of the present invention is based in the disruption of the glucose steady-state.

The disruption of the glucose steady-state can be achieved by different means. In the present invention, the disruption of the glucose steady-state is achieved by reducing extracellular glucose in a single step to a concentration lower than intracellular glucose but different from zero. Such perturbation leads to a rapid decrease towards a new steady-state. Critically, there is an instant in the course of the decay when glucose inside and outside are identical. Because the transporters that mediate glucose uptake into most mammalian cells are equilibrative, that is, they only move glucose when there is a concentration gradient across the plasma membrane, in the moment at which glucose is equilibrated, there will be no net flux through the transporter, or for that matter through simple diffusion or any other passive pathway. Thus eliminated the contribution of permeability, the rate of glucose concentration decrease at glucose equilibrium will be identical to the rate of hexokinase, i.e. the rate of glycolysis at its entry point.

The method of the present invention can be better explained in 6 steps:
First step: Providing a system for the measurement of glucose metabolic rate.
   The system can be cells in isolation, a cell culture, a tissue culture, a mixed cell culture, in tissue explant or a tissue in a living animal. In non-limiting illustrative examples, the system can be a cell culture of astrocytes, neurons, fibroblasts, adipocytes or muscle cells.

Second step: Expressing a suitable glucose sensor in individual cells.
The glucose sensor should be able to monitor the glucose concentration in real-time. Suitable glucose sensors should provide an easy to read signal in a glucose concentration-dependent manner and be insensitive to other molecules commonly present in cells. The expression, presence or degradation of the sensor should not interfere significantly with the cell metabolism. In a non-limiting illustrative example, the glucose sensor can be FLIPglu600µΔ11, described in Patent application WO2007046786.

If the sensor is genetically-encoded, the gene expression can be attained by any genetic-engineering method, for instance plasmid transfer, virus transfer or transgenesis.

Third step: Calibrating the sensor in controlled conditions.
The sensor is calibrated firstly by pre-treating the cells for 30 minutes with 0.5 mM iodoacetic acid to block glycolysis. In the absence of glycolytic flux, the presence of equilibrative GLUT transporters makes the concentration of glucose inside and outside the cell identical. Next, the cells are exposed to increasing concentrations of glucose and the signal given by the sensor is monitored. Sensor signal is plotted versus concentration to estimate the saturation parameters. This procedure is carried out once for each different cell type. For subsequent measurements, the signal is measured transiently in cells in the absence of glucose. This "zero" reading is used together with the aforementioned saturation parameters to transform sensor signal into glucose concentration.

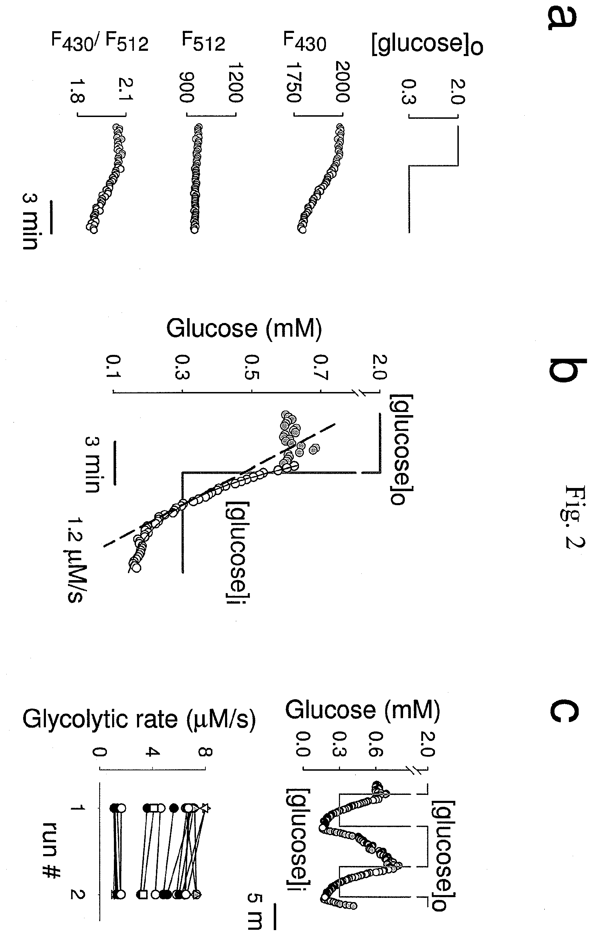

Fourth step: Disrupting the steady-state of glucose entering the cell.
The disruption of the steady-state of glucose is obtained by exposing the extracellular space to a variation in the concentration of glucose (ETM). In a non-limiting illustrative example, the extracellular glucose concentration is lowered from 2 mM to 0.3 mM. This change in extracellular glucose causes a progressive decline in intracellular glucose concentration, from 1 mM to 0.1 mM (Fig. 2b). The assay has been repeated several times, and as illustrated in Fig. 2c, the rate was not affected by a previous measurement, suggesting that the assay itself does not perturb glycolysis. This insensitivity of metabolism to a moderate decrease in intracellular glucose is consistent with the constancy of metabolic flux while hexokinase remains saturated.

Fifth step: Recording the output from the sensor and calculate the corresponding glucose concentration at different times.

The reading from the sensor is registered in time, considering appropriate intervals of time, ranging from 100 ms to 1 minute, for example, every 100 ms, every second, every 10s, every 1 minute. The output from the sensor reading and the calibration curve allows the calculation of glucose concentration at each time point.

Sixth step: Determining the glucose metabolic rate.
The rate of glycolysis is estimated by fitting a monoexponential function to the time course of decay. The metabolic rate is computed from the rate of glucose concentration decrease at the point when the intracellular concentration becomes equal to the extracellular concentration, for instance at 0.3 mM (interrupted line in Fig. 2b).
In a further embodiment, the method can be incorporated in a diagnostic kit for the purposes of metabolic rate measurement in human or animal tissue samples.

The method of the invention can be applied to determine the metabolic rate in any situation it is needed.

Cancer cells are characterized by a substantial increase in their rate of glycolysis, so called a glycolytic phenotype, which is important for their capacity to form metastasis. Pharmacological reversion of the glycolytic phenotype has been shown to cause partial reversal of the cancerous phenotype. In one embodiment, the method can be applied to determine the metabolic rate in a cancer biopsy, thus helping to evaluate the rate of glycolysis in the cells and therefore how aggressive is the cancer and the most appropriate course of action and drugs to be administered. Cancer types that can be subjected to analysis according to the method of the present invention are selected, but not limited to breast cancer, bladder cancer, colon cancer, glioblastoma, lung cancer, hepatocellular carcinoma, gastric cancer, melanoma, thyroid cancer, endometrial cancer, kidney cancer, cervix cancer, pancreatic cancer, esophagus cancer, prostate cancer, brain cancer, ovary cancer, small cell lung cancer, non small cell lung cancer, head and neck cancer, mesothelioma, sarcoma, pediatric malignancies, cholangiocarcinoma.

In other embodiment, the method can be applied in high-throughput manner using a cancer cell line to test the effects of potential anti-cancer drug candidates. Good candidates would be able to decrease the glycolytic rate of the cancer cell line. In particular, the screening of anti-cancer drug candidates would employ the method of the invention in different sets of conditions. Cancerous cell lines suitable for the screening of potential anti-cancer drugs are selected among, but not limited to HeLa cells, Neuro 2A, Caco2, C6, A549, MCF7, PC-3, AGS.

Diabetes mellitus is caused by a decrease in the capacity of muscle cells and adipocytes to metabolize glucose. Hypoglycemic drugs may ameliorate diabetes by increasing the capacity of muscle cells and adipose cells to metabolize glucose. In a further embodiment, the present invention can be applied in a high throughput manner to 3T3-L1 fibroblast and adipocytes, C2C12 myoblasts and myocytes, etc., where a series of potential drug candidates may be tested for its effects in metabolic rate modulation.

In the particular case, where the method can be applied in a high-throughput manner for screening of potential drug candidates, the particular potential drug candidate is added to the culture medium.

### WORKING EXAMPLES

Standard chemicals and tissue culture reagents were from Sigma (St. Louis, MO). Constructs coding for the sensors FLIPglu170n, FLIPglu600µΔ11, FLII¹²Pglu600µΔ6 □have been described previously ^{4, 6, 7}. Plasmids are available through www.addgene.org. Adenoviral vectors Ad FLIPglu600µΔ11 and Ad FLII¹²Pglu600µΔ6□ were custom made by Vector Biolabs.

### Animals, cell culture and tissue slices

Animals used were mixed F1 male mice (C57BL/6J x CBA/J), kept in an animal room under SPF conditions at a room temperature of 20 ± 2 °C, in a 12/12 h light/dark cycle with free access to food and water. All experiments were approved by the Centro de Estudios Cientificos Animal Care and Use Committee. Mixed cortical cultures of neuronal and glial cells were prepared from 1-3 day-old neonatal mice as described in Loaiza et al. ¹⁹, except that N1-N2/MEM was replaced by B27-supplemented Neurobasal medium (Gibco). Cultures were maintained at 37 °C in a humidified atmosphere of 5% CO₂. At days 5-7, cultures in 35 mm dishes were transfected with 5 µg plasmid DNA using Lipofectamine 2000 (Gibco) or alternatively, exposed to 5 x 10⁶ PFU of adenoviral vector. Adenoviral vectors showed a very high selectivity for astrocytes over neurons, with a ratio > 100. Cell lines were obtained from the ATCC. 3T3-L1 fibroblasts were maintained in Dulbecco's Modified Eagle Medium (DMEM) supplemented with 10% fetal calf serum, 2.5 µg/ml amphotericin B and 100 U/ml penicillin/streptomycin and differentiated into adipocytes as described ²⁵ using 5 µg/ml insulin. C2C12 myoblasts and Hela cells were maintained in DMEM supplemented with 10% fetal bovine serum, 2.5 µg/ml amphotericin B and 100 U/ml penicillin/streptomycin. 3T3-L1 fibroblast and adipocytes growing in 10 cm dishes were electroporated with 15 µg plasmid DNA using the BioRad Gene Pulser XCell. HeLa cells in 35 mm dishes were transfected with 5 µg plasmid DNA using Lipofectamine 2000.

Hippocampal slices (200 µm thick) were prepared with a Vibratome 1000 Plus (Warner Instruments) from 15 day-old mice by following standard procedures ²⁶. Briefly, brains were immersed in ice-cold aCSF of the following composition (in mM): 125 NaCl, 2.5 KCI, 1.25 NaH₂PO₄ 2.5 MgCl2, 0.5 CaCl₂, 25 glucose, 26 NaHCO₃, that had been previously bubbled with 5% CO₂/95% O₂ for 1 hour to reach pH 7.4. Hippocampi were dissected from coronal slices and incubated for 1-2 hrs in cold dissection medium. The slices were then transferred to 35 mm Petri dishes and cultured at 37 °C in a humidified atmosphere of 5% CO₂ for 5-6 days in 50 % (MEM containing 6.5 g/L glucose, 23 mM HEPES and 26 mM NaHCO₃), 25 % fetal bovine serum, 25 % Hank's solution containing 6.5 g/L glucose, 0.3 % glutamine, 1 mM sodium pyruvate, non-essential amino acids, N2 supplement (Invitrogen), 100 U / ml penicillin and 100 µg / ml streptomycin. Slices were infected for 24 hrs with 50 x 10⁶ PFU of the adenoviral vectors at day 2 of culture. For immunohistochemistry, the slices were fixed overnight in 4% paraformaldehyde in phosphate buffered saline (PBS). After extensive washing with PBS, slices were incubated overnight at 4 oC with rabbit 1:500 anti-cow GFAP antisera (Dako). After further washing with PBS, the tissue was incubated overnight at 4 °C with 1:500 Alexa Fluor 568 goat anti-rabbit IgG (Molecular Probes). Slices were mounted on DakoCytomation medium (Dako) and imaged with a Pascal 5 Zeisss confocal microscope at 488 excitation/505-550 emission (for YFP) and 543 excitation/>580 nm emission (for Alexa Fluor 568).

### Glucose and volume measurements

Experiments were carried out at room temperature (22-25 °C). Cultured cells were imaged in HEPES-buffered saline containing (in mM): 136 NaCl, 3 KCI, 1.25 CaCl₂, 1.25 MgSO_{4,} 1-2 glucose, 2 sodium lactate, 10 HEPES, pH 7.4 or in 95% 02/5% CO₂ -gassed buffer of the following composition (in mM): 112 NaCl, 3 KCI, 1.25 CaCl₂, 1.25 MgCl₂, 1-2 glucose, 2 sodium lactate, 10 HEPES, 24 NaHCO₃, pH 7.4. Brain slices were superfused with a 95% O₂/5% CO₂ -gassed buffer containing (in mM): 126 NaCl, 3 KCI, 1.25 NaH₂PO_{4,} 1.25 CaCl₂, 1.25 MgCl₂, 2-3 glucose, 1 sodium lactate, 26 NaHCO₃, pH 7.4. When using higher K+ concentrations, NaCl was adjusted to maintain isotonicity. Cultures and slices were imaged with an Olympus IX70 inverted microscope equipped with a 40x oil-immersion objective, a Cairn monochromator with Optosplit (Faversham, UK), and a Hamamatsu Orca camera (Hamamatsu City, Japan) controlled by Kinetics software. For FRET measurements, CFP was excited at 430 nm for 400 ms. Because of the very high FRET efficiency of FLII12Pglu600µΔ6□□, which results in low CFP signal⁷, we opted for not using the CFP signal in our protocol. Instead, YFP/citrine was excited for 50 ms, which provided a sugar-insensitive denominator for the FRET ratio. The ratio between YFP/Citrine excited by FRET (at 430 nm) and excited directly (at 512 nm) was calibrated by exposing pH-clamped cells to increasing glucose concentrations in the presence of the glycolytic blocker iodoacetic acid. Figure 8 includes the calibration curves for astrocytes, fibroblasts, myoblasts and HeLa cells. For neurons and adipocytes, whose low glucose permeability made calibration very difficult, we used the calibration curves obtained in astrocytes and fibroblasts, respectively. Relative cell volume was measured using a Pascal 5 Zeisss confocal microscope (optical section < 2 µm; 488 nm excitation/505-550 nm emission) in cells ester-loaded with calcein at 0.5 µM for 30 min ²⁷. Exposure to anisosmotic solutions showed that over 90% of the calcein fluorescence was volume-sensitive.

### Metabolic rate determination in different systems

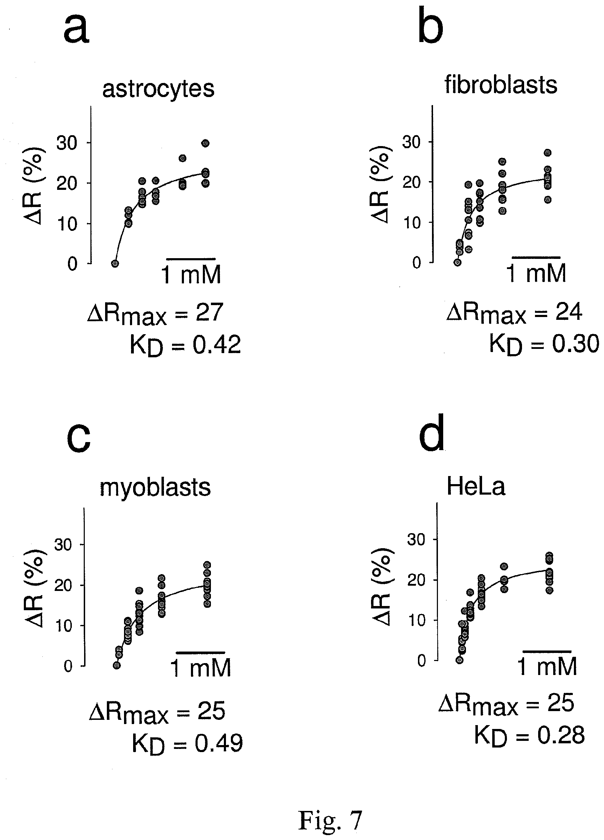

Determination of metabolic rate in cultured astrocytes
Intracellular glucose was measured in real-time with a DNA-encoded FRET glucose nanosensor ^{4, 6, 7} that was calibrated *in situ* (Figure 7). In the presence of 2 mM extracellular glucose, cultured astrocytes maintained a steady-state intracellular glucose concentration averaging 0.63 ± 0.30 mM (n=100 cells in twenty two experiments). The intracellular glucose concentration varied greatly from cell to cell, ranging from 0.2 to 1.9 mM, showing that the balance between glucose permeability and glycolysis is not fixed.

The disruption of the steady-state was obtained by lowering extracellular glucose to 0.3 mM. Lowering extracellular glucose caused a progressive decline in intracellular glucose concentration (Fig. 2b). The rate of glycolysis was estimated by fitting a monoexponential function to the time course of decay and computing the instantaneous slope at 0.3 mM (interrupted line in Fig. 2b) as a metabolic rate of 1.2 µM/s. Next, the assay was repeated several times. As illustrated in Fig. 2c, the rate was not affected by a previous measurement, suggesting that the assay itself does not perturb glycolysis. This insensitivity of metabolism to a moderate decrease in intracellular glucose is consistent with the constancy of metabolic flux while hexokinase remains saturated.

Further to the example above, adding a GLUT inhibitor to the extracellular space was also tested.

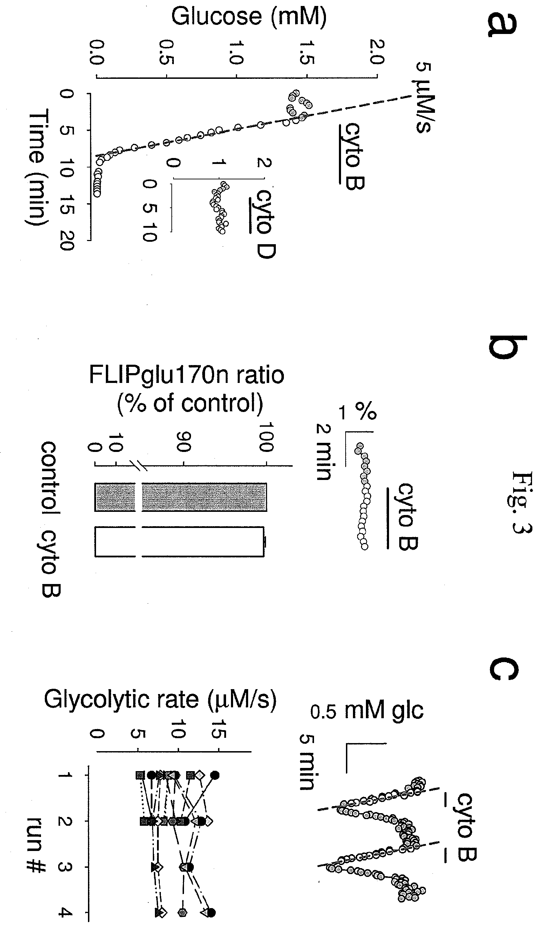

The most widely used inhibitor of the glucose transporter is cytochalasin B, which blocks the transporter isoforms present in most cell types, GLUT1, GLUT3 and GLUT4, with a Kᵢ of about 1 µM or lower ¹⁰. Exposure of astrocytes to the inhibitor resulted in a linear decrease in the concentration of glucose (Fig. 3a). In the continuous presence of cytochalasin B, the concentration of glucose reached levels indistinguishable from zero, demonstrating the high degree of transport inhibition achieved. The average rate of glycolysis in cultured astrocytes was 2.0 ± 0.3 µM/s (n=120 cells in thirty experiments) and showed high heterogeneity, with differences of several-fold even inside a microscopic field (data not shown). Several control experiments were designed to check the validity of the disruption of steady-state by adding a GLUT inhibitor. In addition to inhibit the glucose transporter, cytochalasin B affects the actin cytoskeleton, a potential source of interference that can be controlled for with cytochalasin D, a structural analog that targets the cytoskeleton but not the glucose transporter ¹⁰. The experiment illustrated in Fig 3a (inset) supports the specificity of cytochalasin B by showing that cytochalasin D did not affect the concentration of glucose. To control for direct effects of cytochalasin B on the sensor, the inhibitor was applied to galactose-equilibrated cells expressing FLIPglu170n. This very high affinity version of the sensor is saturated in the presence of physiological glucose concentrations ⁶, so that any change in its behavior can be unambiguously ascribed to an artifact. As illustrated in Fig. 3b, cytochalasin B did not change the ratio of FLIPglu170n emission intensity, showing that there is no direct effect of the inhibitor on the sensor. Disruption of the steady-state by using a GLUT inhibitor is highly reproducible, confirming that a moderate decrease in intracellular glucose does not affect metabolism (Fig. 3c). Phloretin, a structurally unrelated inhibitor of the glucose transporter, gave a glycolytic rate of 1.7 ± 0.3 µM/s which was not significantly different from the glycolytic rate obtained with cytochalasin B (1.8 ± 0.3 M/s; n=20 cells in three paired experiments).

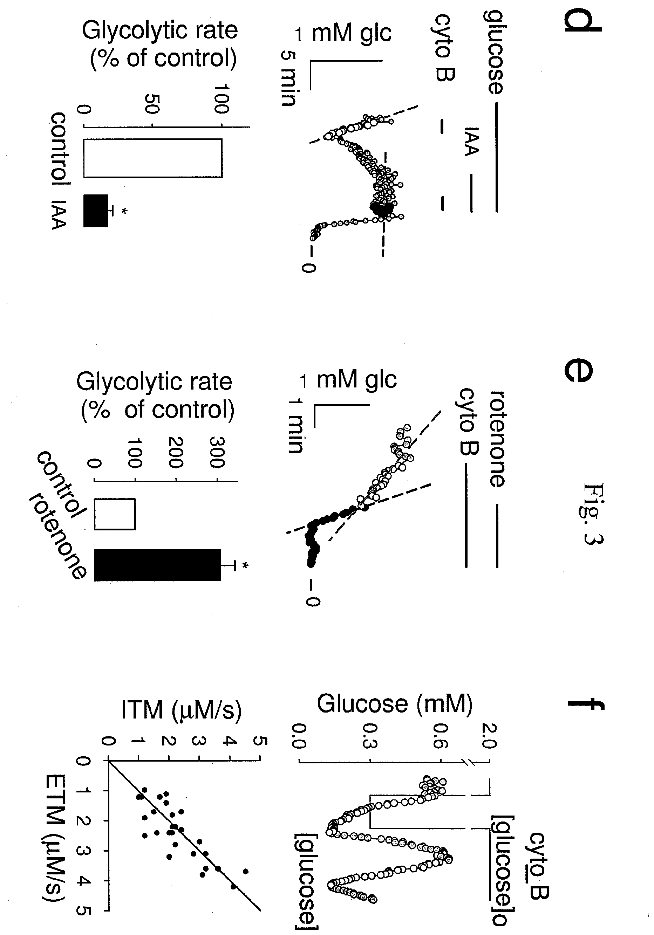

A comparison using the protocol illustrated in Fig. 3f was used, and as it was expected, their outputs were highly correlated. This shows that the difference in the average metabolic rates obtained by altering the extracellular glucose concentration of 1,2 µM/s compared to the metabolic rate obtained by adding a GLUT inhibitor of 2,0 µM/s is not due to differences between the output of the method but to cell batch variation.

### Temporal resolution: acute activation of astrocytic glycolysis by neuronal signals

Typical acquisition times in deoxyglucose uptake, deoxyglucose autoradiography and FDG-PET scanning are longer than 10 minutes, but the metabolic changes that characterize the brain tissue develop over seconds. For instance, neuronal activity is accompanied by sub-second shifts in mitochondrial redox potential ³, followed in seconds by a rise in interstitial lactate ¹¹, which is thought to play key roles in fast neurovascular and neurometabolic coupling ¹²⁻¹⁴. Both the identity of the cell type responsible for the lactate surge (i.e. neurons versus astrocytes) and the identity of the local signals that link electrical activity to metabolic activation are controversial ^{13, 15, 16 9}, which is partly explained by the inability of deoxyglucose to monitor events in the range of seconds. Taking advantage of capability of following glycolytic activation in real-time, cultured astrocytes were exposed simultaneously to glutamate and K⁺, mediators that are co-released by neurons at the excitatory synapse. The data, summarized in Figure 4, show that the neuronal signals trigger a robust and rapid increase in the rate of glycolysis in astrocytes. Remarkably, the activation was even stronger than the activation achieved by mitochondrial poisoning (Fig. 3e). A control experiment showed that the fast fall in glucose concentration observed in response to glutamate/K⁺ can not be explained by sugar dilution during cell swelling (Figure 8). By showing for the first time that astrocytic glycolysis can be activated by neuronal signals in the range of seconds, these results suggest that astrocytes are responsible for the lactate surge observed during neuronal activation and lend fresh support to their proposed role in neurometabolic and neurovascular coupling. This level of temporal resolution cannot be obtained with any of the currently available methods or sensors, giving to the present invention a great advantage over them.

### Spatial resolution: neurons versus astrocytes

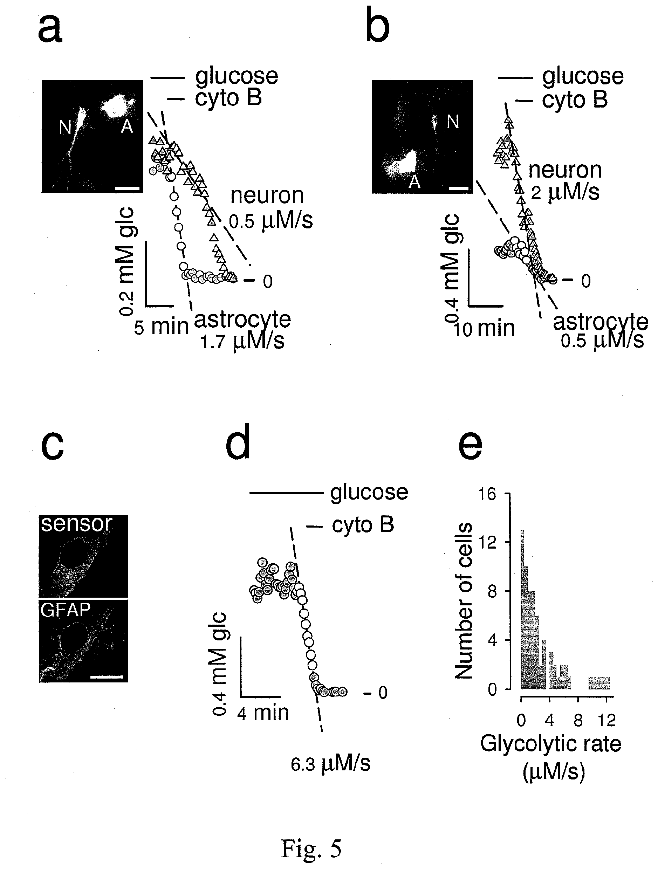

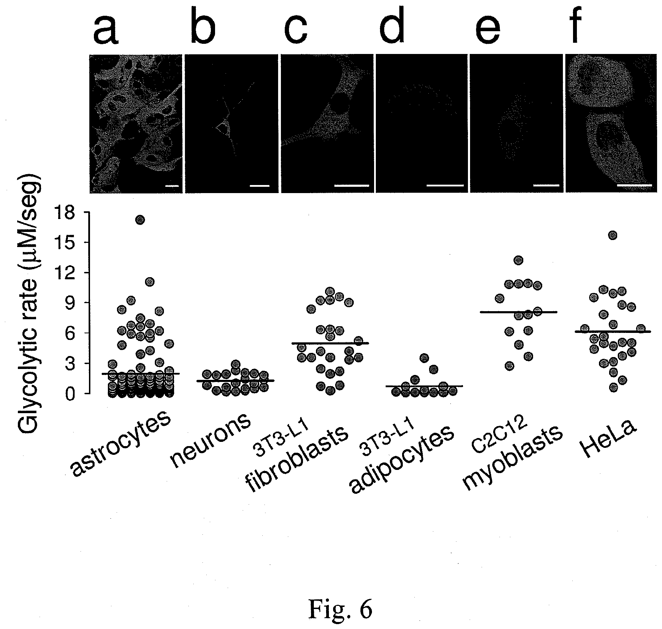

The brain tissue is known in great detail both anatomically and functionally, but its energy usage has not been mapped at high spatial resolution. Each millimeter-sized voxel of an autoradiograph or PET scan is populated by scores of different neuronal and glial types, each cell consuming fuel at a specific rate which is currently not accessible. A way forward is suggested by the two experiments illustrated in Fig. 5a, which shows simultaneous measurement of the glycolytic rate in a neuron and a neighboring astrocyte. We observed great variability of the basal metabolic rate in both cell types but on average the faster cell was the astrocyte (see also Fig. 6). These results in cultured cells do not predict the situation in the brain tissue but given that the FRET nanosensor may be targeted to specific cell types *in vivo* by means of viral vectors or transgenesis, this kind of measurement may eventually be carried out in the brain tissue *in vivo.* As an initial step in that direction, the sensor was expressed in hippocampal slices using an adenoviral vector. Figure 5b shows colocalization between the sensor and the glial protein GFAP, consisting with the preferential targeting of glial cells by these vectors. Similar to that observed in cultured cells, Figure 5c-d show that the metabolic rate in slices was highly heterogeneous, with an average of 2.8 ± 0.4 µM/s (n=68 cells in twenty five slices, ranging from 0.02 to 12 µM/s).

### Other cell types

The metabolic rate could also be measured in other cell types, demonstrating the general applicability of the method (Fig. 6). The results were consistent with previous measurements using radioactive techniques for high rates were observed in undifferentiated and tumor cells (C2C12 myoblasts and HeLa cells) and differentiation of 3T3-L1 fibroblasts into adipocytes led a marked decrease in metabolism. Whereas radioactive techniques and the current methods give similar results, the reliance of the latter on fluorescence should allow their adaptation for the purposes of high-throughput analysis. Moreover, as illustrated in Fig. 6, cultured cell lines showed a high degree of metabolic heterogeneity, perhaps related to cell cycle or other sources of variation, which now seem easier to address.

The working examples described in the present specification are presented with the intention to illustrate the present invention, and are not to be interpreted as a limitation of the scope of the invention.

The present invention introduces new strategies for the measurement of the metabolic rate, whose common rationale is the isolation of glucose phosphorylation by eliminating the contribution of the glucose transporters. The method of the invention offers temporal resolution of seconds and spatial resolution of micrometers, comparing favorably with the method based on 2-deoxyglucose, which offer temporal resolution of minutes and spatial resolution of millimeters. Another important advantage of the present invention is the use of glucose itself, as opposed to glucose analogs, whose handling by hexokinase and posterior fates are usually unknown. One of these analogs, 2-NBDG, the fluorescent version of deoxyglucose, can provide cellular resolution but its rate of uptake is not directly informative about metabolic rate because is orders of magnitude lower than that of glucose and highly sensitive to the properties of the glucose transporter ¹⁸⁻²⁰. In previous applications, the FRET glucose nansosensor had been used to measure glucose concentration ^{4, 6, 7}, which depends on both the activity of the glucose transporter and that of hexokinase, and therefore does not inform about metabolic flux. The current approaches circumvent the ambiguity of glucose concentration measurements by providing a direct readout of metabolic flux, without interference from transport properties.

The present invention uses a FRET glucose nanosensor, but any other technique capable of real-time measurement of intracellular glucose concentration may be used for the same purpose. The method of the invention offers cellular resolution and is reversible, presenting different strengths and weaknesses that make them complementary. For example, the method does not require an inhibitor and therefore can be applied to hepatocytes and pancreatic beta-cells, which are rich in GLUT2 and therefore relatively insensitive to cytochalasin B and phloretin. The temporal resolution of the method of the invention is much better and only limited by the time required for data acquisition, usually less the 1 second with high-end setups, providing an extended window of measurement that is ideal for before-and-after kind of experiments such as shown in Fig. 5. Although such was not observed to be the case for cytochalasin B in astrocytes, control experiments such as those in Fig. 3 are advised when investigating a new cell type.

In addition to the method of the invention claimed in this application, two original observations were made. Firstly, it was possible for the first time to observe that astrocytic glycolysis can be activated within seconds by neuronal signals. Previous measurements of deoxyglucose uptake over 20 minutes had given conflicting results ^{13, 15}, but the present method, with its temporal resolution of seconds, was able to show that astrocytic glycolysis is highly sensitive to mediators that are released at the excitatory synapse. The strength and speed of the glycolytic activation observed in astrocytes provides a mechanistic explanation for the fall in glucose concentration and the surge in lactate concentration that can be detected in the brain tissue 5-20 seconds after the onset of neuronal activation ^{11, 21-24}, highlighting the role of astrocytes in metabolic coupling and blood flow regulation in the brain ^{12, 14}. The improved spatial resolution of the method allowed for the first time to monitor the glycolytic rate of a neuron lying on astrocytes. Given the inherent limitations of isotopic measurements, previous work had compared these cells as cultured in isolation, but this is not ideal because both cell types require each other for their proper differentiation and function. Our results in co-culture show high cell-to-cell variability, but on average astrocytes were found to metabolize glucose faster than neurons. Astrocytes could also be studied in brain slices, suggesting that in the near future it will be possible to measure metabolic rates of both cells types in the tissue, in slices and *in vivo.*

In order to demonstrate the use of these methods in other cell types, we chose fibroblasts, adipocytes and myoblasts, cells that are widely used for research and drug-screening. The hypoglycemic effect of insulin is explained by an increase in the metabolic rate of adipocytes and muscle cells, a process that becomes defective in diabetes; we envisage that the current methods may be useful for basic and applied research related to this disease. Very high metabolic rates were observed in HeLa, an epithelial cell line of ample use in cancer research. The enhanced glycolytic rate observed in tumor cells, i.e. the Warburg effect, is widely exploited for the purposes of diagnosis and staging by means of FDG-PET scanning, and has recently proposed to play a pathogenic role in cancer progression ¹. Thus, the adaptability of the current fluorescent techniques for high-throughput screening and the metabolic heterogeneity evidenced at the single cell level may be instrumental for basic and applied research on cancer.

### References

1. Vander Heiden, M. G., Cantley, L. C., & Thompson, C. B. Understanding the Warburg effect: the metabolic requirements of cell proliferation. Science. 324, 1029-1033 (2009).
2. Yeluri, S., Madhok, B., Prasad, K. R., Quirke, P., & Jayne, D. G. Cancer's craving for sugar: an opportunity for clinical exploitation. J. Cancer Res. Clin. Oncol. 135, 867-877 (2009).
3. Brennan, A. M., Connor, J. A., & Shuttleworth, C. W. NAD(P)H fluorescence transients after synaptic activity in brain slices: predominant role of mitochondrial function. J. Cereb. Blood Flow Metab. 26, 1389-1406 (2006).
4. Deuschle, K. et al. Construction and optimization of a family of genetically encoded metabolite sensors by semirational protein engineering. Protein Sci. 14, 2304-2314 (2005).
5. Berg, J., Hung, Y. P., & Yellen, G. A genetically encoded fluorescent reporter of ATP:ADP ratio. Nat. Methods. 6, 161-166 (2009).
6. Fehr, M., Lalonde, S., Lager, I., Wolff, M. W., & Frommer, W. B. In vivo imaging of the dynamics of glucose uptake in the cytosol of COS-7 cells by fluorescent nanosensors. J. Biol. Chem. 278, 19127-19133 (2003).
7. Takanaga, H., Chaudhuri, B., & Frommer, W. B. GLUT1 and GLUT9 as major contributors to glucose influx in HepG2 cells identified by a high sensitivity intramolecular FRET glucose sensor. Biochim. Biophys. Acta. 1778, 1091-1099 (2008).
8. Wilson, J. E. Isozymes of mammalian hexokinase: structure, subcellular localization and metabolic function. J. Exp. Biol. 206, 2049-2057 (2003).
9. Barros, L. F. & Deitmer, J. W. Glucose and lactate supply to the synapse. Brain Res. Rev.(2009).
10. Carruthers, A. Facilitated diffusion of glucose. Physiol Rev. 70, 1135-1176 (1990).
11. Hu, Y. & Wilson, G. S. A temporary local energy pool coupled to neuronal activity: fluctuations of extracellular lactate levels in rat brain monitored with rapid-response enzyme-based sensor. J. Neurochem. 69, 1484-1490 (1997).
12. Magistretti, P. J. Role of glutamate in neuron-glia metabolic coupling. Am. J. Clin. Nutr. 90, 875S-880S (2009).
13. Pellerin, L. et al. Activity-dependent regulation of energy metabolism by astrocytes: an update. Glia. 55, 1251-1262 (2007).
14. Gordon, G. R., Choi, H. B., Rungta, R. L., Ellis-Davies, G. C., & MacVicar, B. A. Brain metabolism dictates the polarity of astrocyte control over arterioles. Nature. 456, 745-749 (2008).
15. Dienel, G. A. & Cruz, N. F. Nutrition during brain activation: does cell-to-cell lactate shuttling contribute significantly to sweet and sour food for thought? Neurochem. Int. 45, 321-351 (2004).
16. Mangia, S., Simpson, I. A., Vannucci, S. J., & Carruthers, A. The in vivo neuron-to-astrocyte lactate shuttle in human brain: evidence from modeling of measured lactate levels during visual stimulation. J. Neurochem. 109 Suppl 1, 55-62 (2009).
17. Hewett, J. A. Determinants of regional and local diversity within the astroglial lineage of the normal central nervous system. J. Neurochem. 110, 1717-1736 (2009).
18. Yamada, K., Saito, M., Matsuoka, H., & Inagaki, N. A real-time method of imaging glucose uptake in single, living mammalian cells. Nat. Protoc. 2, 753-762 (2007).
19. Loaiza, A., Porras, O. H., & Barros, L. F. Glutamate triggers rapid glucose transport stimulation in astrocytes as evidenced by real-time confocal microscopy. J. Neurosci. 23, 7337-7342 (2003).
20. Barros, L. F. et al. Preferential transport and metabolism of glucose in Bergmann glia over Purkinje cells: a multiphoton study of cerebellar slices. Glia 57, 962-970 (2009).
21. Silver, I. A. & Erecinska, M. Extracellular glucose concentration in mammalian brain: continuous monitoring of changes during increased neuronal activity and upon limitation in oxygen supply in normo-, hypo-, and hyperglycemic animals. J. Neurosci. 14, 5068-5076 (1994).
22. Hu, Y. & Wilson, G. S. Rapid changes in local extracellular rat brain glucose observed with an in vivo glucose sensor. J. Neurochem. 68, 1745-1752 (1997).
23. Mangia, S. et al. The aerobic brain: lactate decrease at the onset of neural activity. Neuroscience. 118, 7-10 (2003).
24. Caesar, K. et al. Glutamate receptor-dependent increments in lactate, glucose and oxygen metabolism evoked in rat cerebellum in vivo. J. Physiol. 586, 1337-1349 (2008).
25. Nie, Y. & Wong, C. Suppressing the activity of ERRalpha in 3T3-L1 adipocytes reduces mitochondrial biogenesis but enhances glycolysis and basal glucose uptake. J. Cell Mol. Med.(2008)*.*
26. Ridoux, V., Robert, J., Perricaudet, M., Mallet, J., & Le Gal La, S. G. Adenovirus mediated gene transfer in organotypic brain slices. Neurobiol. Dis. 2, 49-54 (1995).
27. Barros, L. F. Measurement of sugar transport in single living cells. Pflugers Arch. 437, 763-770 (1999).
28. Barros, L. F., Bittner, C. X., Loaiza, A., & Porras, O. H. A quantitative overview of glucose dynamics in the gliovascular unit. Glia. 55, 1222-1237 (2007).

## Claims

1. An in-vitro method for the measurement of glucose metabolic rate with high temporal resolution, comprising the steps of:
a. providing single cells or cell populations, cells in suspension or adherent or in a cell culture, a tissue culture, a mixed cell culture, a tissue explant, for the measurement of glucose metabolic rate;
b. expressing a glucose sensor in individual cells;
c. calibrating the sensor in controlled conditions;
d. disrupting the steady-state of glucose entering the cell by lowering extracellular glucose to a concentration lower than the intracellular concentration during the initial steady-state;
e. recording the output from the sensor at intervals of time from 100 ms to 1 minute and calculating the corresponding glucose concentration at different times; and
f. determining the glucose metabolic rate by fitting a monoexponential function to the time course of decay and wherein the metabolic rate is computed from the rate of glucose concentration decrease at the point when the intracellular concentration becomes equal to the extracellular concentration.

2. The method of claim 1, wherein the glucose sensor of step (b) is a fluorescent sensor.

3. The method of claim 2, wherein the glucose sensor is FLIPglu600µΔ11.

4. The method of claim 1, wherein the calibrating of step (c) is performed by pre-treating the cells for 30 minutes with 0.5 mM iodoacetic acid to block glycolysis.

5. The method of claim 4, further comprising:
exposing the cells to increasing concentrations of glucose and monitoring a signal given by the sensor; and
plotting the sensor signal versus glucose concentration to estimate saturation parameters for each different cell type.

6. The method of claim 5, further comprising:
measuring the signal transiently in cells in the absence of glucose to form a zero reading; and
transforming the sensor signal with the zero reading and saturation parameters into glucose concentration.

7. An in-vitro method for the determination of cancer staging, comprising determining the glucose metabolic rate of a sample of cancerous tissue according to the method of claim 1.

8. The method of claim 7, wherein the cancerous tissue is breast cancer, bladder cancer, colon cancer, glioblastoma, lung cancer, hepatocellular carcinoma, gastric cancer, melanoma, thyroid cancer, endometrial cancer, kidney cancer, cervix cancer, pancreatic cancer, esophagus cancer, prostate cancer, brain cancer, ovary cancer, small cell lung cancer, non small cell lung cancer, head and neck cancer, mesothelioma, sarcoma, pediatric malignancies, cholangiocarcinoma.

9. An in-vitro method for screening potential drugs, comprising determining the glucose metabolic rate of a model cell culture exposed to the potential drug according to the method of claim 1.

10. The method of claim 9, wherein the model cell culture is a culture of 3T3-L1 fibroblast and adipocytes, C2C12 myoblasts and myocytes.

11. The method of claim 9, wherein the model cell culture is a culture of HeLa cells, Neuro 2A, Caco2, C6, A549, MCF7, PC-3, and AGS.

## Patentansprüche

1. In-vitro-Verfahren zur Messung der Verstoffwechselungsrate von Glucose mit hoher zeitlicher Auflösung, umfassend folgende Schritte:
a. das Bereitstellen von einzelnen Zellen oder Zellpopulationen, Zellen in Suspension oder adhärenten Zellen oder in einer Zellkultur, einer Gewebekultur, einer gemischten Zellkultur, einem Gewebeexplantat, zur Messung der Verstoffwechselungsrate von Glucose;
b. das Exprimieren eines Glucosesensors in einzelnen Zellen;
c. das Kalibrieren des Sensors unter kontrollierten Bedingungen;
d. das Stören des stationären Zustands der in die Zelle eindringenden Glucose, durch das Senken der extrazellulären Glucose bis zu einer Konzentration kleiner als die intrazelluläre Konzentration während des anfänglichen stationären Zustands;
e. das Erfassen der Ausgabe aus dem Sensor in Zeitbereichen von 100 ms bis 1 Minute und das Berechnen der entsprechenden Glucosekonzentration zu unterschiedlichen Zeiten; und
f. das Bestimmen der Verstoffwechselungsrate von Glucose durch die Anpassung einer monoexponentiellen Funktion an den Zeitablauf des Zerfalls und wobei die Verstoffwechselungsrate aus der Rate der Glucosekonzentrationssenkung zum Zeitpunkt errechnet wird, in welchem die intrazelluläre Konzentration der extrazellulären Konzentration gleich wird.

2. In-vitro-Verfahren nach Anspruch 1, wobei der Glucosesensor aus Schritt (b) ein fluoreszierender Sensor ist.

3. In-vitro-Verfahren nach Anspruch 2, wobei der Glucosesensor FLIPglu600µΔ11 ist.

4. In-vitro-Verfahren nach Anspruch 1, wobei das Kalibrieren aus Schritt (c) mittels der Vorbehandlung der Zellen 30 Minuten lang mit 0,5 mM Jodessigsäure durchgeführt wird, um die Glykolyse zu blockieren.

5. In-vitro-Verfahren nach Anspruch 4, zusätzlich umfassend:
das Aussetzen der Zellen gegenüber zunehmenden Glucosekonzentrationen und das Überwachen eines vom Sensor abgegebenen Signals; und
das Auftragen des Sensorsignals gegenüber der Glucosekonzentration, um Sättigungsparameter für jeden unterschiedlichen Zelltyp zu schätzen.

6. In-vitro-Verfahren nach Anspruch 5, zusätzlich umfassend:
das transiente Messen des Signals in Zellen in Abwesenheit von Glucose um eine Nullablesung zu bilden; und
das Umwandeln des Sensorsignals mit der Nullablesung und Sättigungsparametern in Glucosekonzentration.

7. In-vitro-Verfahren zur Bestimmung der Krebsstadienbestimmung, umfassend das Bestimmen der Verstoffwechselungsrate von Glucose einer Probe von Krebsgewebe gemäß dem Verfahren nach Anspruch 1.

8. In-vitro-Verfahren nach Anspruch 7, wobei das Krebsgewebe aus Brustkrebs, Blasenkrebs, Kolonkrebs, Glioblastom, Lungenkrebs, Leberzellkarzinom, Magenkrebs, Melanom, Schilddrüsenkrebs, Gebärmutterschleimhautkrebs, Nierenkrebs, Gebärmutterhalskrebs, Bauchspeicheldrüsenkrebs, Speiseröhrenkrebs, Prostatakrebs, Hirnkrebs, Eierstockkrebs, kleinzelligem Lungenkrebs, nicht-kleinzelligem Lungenkrebs, Kopf-Hals-Krebs, Mesotheliom, Sarkom, pädiatrischen Malignitäten, Cholangiokarzinom ist.

9. In-vitro-Verfahren zur Untersuchung von potentiellen Arzneimitteln, umfassend das Bestimmen der Verstoffwechselungsrate von Glucose einer Modellzellkultur, welche dem potentiellen Arzneimittel ausgesetzt wird gemäß dem Verfahren nach Anspruch 1.

10. In-vitro-Verfahren nach Anspruch 9, wobei die Modellzellkultur eine Kultur aus 3T3-L1-Fibroblasten und Adipozyten, C2C12-Myoblasten und Myozyten ist.

11. In-vitro-Verfahren nach Anspruch 9, wobei die Modellzellkultur eine Kultur aus HeLa-Zellen, Neuro 2A, Caco2, C6, A549, MCF7, PC-3 und AGS ist.

## Revendications

1. Méthode *in vitro* de mesure du taux métabolique du glucose à une résolution temporelle élevée, comprenant les étapes de :
a. fournir des cellules individuels ou des populations de cellules, des cellules en suspension ou adhérentes ou dans une culture cellulaire, une culture tissulaire, une culture cellulaire mixte, un explant tissulaire, pour mesurer le taux métabolique du glucose ;
b. exprimer un capteur de glucose dans des cellules individuelles ;
c. calibrer le capteur dans des conditions contrôlées ;
d. interrompre l'état stable de glucose entrant dans la cellule en réduisant le glucose extracellulaire à une concentration inférieure à la concentration intracellulaire pendant l'état stable initial ;
e. enregistrer la sortie à partir du capteur à des intervalles de temps allant de 100 ms à 1 minute et calculer la concentration de glucose correspondante à différents temps ; et
f. déterminer le taux métabolique du glucose en ajustant une fonction mono-exponentielle au cours du temps de dégradation et dans laquelle le taux métabolique est calculé à partir du taux de réduction de la concentration de glucose au point où la concentration intracellulaire devient identique à la concentration extracellulaire.

2. Méthode selon la revendication 1, dans laquelle le capteur de glucose de l'étape (b) est un capteur fluorescent.

3. Méthode selon la revendication 2, dans laquelle le capteur de glucose est FLIPglu600µΔ11.

4. Méthode selon la revendication 1, dans laquelle le calibrage de l'étape (c) est réalisé par prétraitement des cellules pendant 30 minutes avec de l'acide iodoacétique 0,5 mM pour bloquer la glycolyse.

5. Méthode selon la revendication 4, comprenant en outre :
exposer les cellules à des concentrations croissantes de glucose et monitorer un signal émis par le capteur ; et
tracer le signal de capteur par rapport à la concentration de glucose pour estimer les paramètres de saturation pour chaque type de cellule différent.

6. Méthode selon la revendication 5, comprenant en outre :
mesurer le signal transitoirement dans les cellules en absence de glucose pour former une lecture de zéro ; et
transformer le signal de capteur avec la lecture de zéro et les paramètres de saturation en une concentration de glucose.

7. Méthode *in vitro* de détermination de la stadification du cancer, comprenant la détermination du taux métabolique du glucose d'un échantillon de tissu cancéreux selon la méthode de la revendication 1.

8. Méthode selon la revendication 7, dans laquelle le tissu cancéreux est du cancer du sein, cancer de la vessie, cancer du colon, un glioblastome, cancer du poumon, un carcinome hépatocellulaire, cancer gastrique, un mélanome, cancer de la tyroïde, cancer de l'endomètre, cancer du rein, cancer du col de l'utérus, cancer du pancréas, cancer de l'oesophage, cancer de la prostate, cancer du cerveau, cancer des ovaires, cancer du poumon à petites cellules, cancer du poumon non à petites cellules, cancer de la tête et du cou, un mésothéliome, un sarcome, des malignités pédiatriques, un cholangiocarcinome.

9. Méthode *in vitro* de criblage de médicaments potentiels, comprenant la détermination du taux métabolique du glucose d'une culture cellulaire modèle exposée au médicament potentiel selon la méthode de la revendication 1.

10. Méthode selon la revendication 9, dans laquelle la culture cellulaire modèle est une culture d'adipocytes et de fibroblaste 3T3-L1 et de myocytes et de myoblastes C2C12.

11. Méthode selon la revendication 9, dans laquelle la culture cellulaire modèle est une culture de cellules HeLa, Neuro 2A, Caco2, C6, A549, MCF7, PC-3 et AGS.
